# EUROPÄISCHE PATENTSCHRIFT

(11) **EP 1 666 077 B1**
(45) Veröffentlichungstag und Bekanntmachung des Hinweises auf die Patenterteilung: **12.12.2012**
(21) Anmeldenummer: 05022765.1
(22) Anmeldetag: 19.10.2005
(51) Int. Cl.: A61L 31/16, A61L 31/18

(54) **Chirurgisches Trägermaterial mit Silberpartikeln, medizintechnisches Produkt enthaltend das Trägermaterial und Verfahren zur Detektion des Trägermaterials sowie von Adhäsionen**
Surgical carrier material with silver particles, medical product containing the carrier material and method for detection of the carrier material as well as of adhesions
Matériau de support avec des particules d'argent, produit médical contenant ce matériau de support et méthode pour la détection du matériau de support ainsi que d'adhésions

(30) Priorität: 20.10.2004 DE 102004052203
(43) Veröffentlichungstag der Anmeldung: 07.06.2006
(73) Patentinhaber: Aesculap AG, 78532 Tuttlingen (DE)
(72) Erfinder: Tümmler, Hanns-Peter, 78532 Tuttlingen (DE); Kozak, Josef, 78532 Tuttlingen (DE); Odermatt, Erich, CH-8200 Schaffhausen (CH); Abele, Wolfgang, 78532 Tuttlingen (DE); Weis, Christine, 78532 Tuttlingen (DE)
(74) Vertreter: Patentanwälte Ruff, Wilhelm, Beier, Dauster & Partner

(56) Entgegenhaltungen:
- WO-A-01/56499
- WO-A-02/096474

## Beschreibung

In der Chirurgie besteht bei jedem invasiven Eingriff, insbesondere im Bauchraum, die Gefahr von Adhäsionen in der Zeit nach der Operation. Durch diese postoperativen Adhäsionen im Bauchraum, beispielsweise zwischen Darmabschnitten oder anderen Organen und der Bauchwand, kann es zur Beeinträchtigung der Funktionalität des jeweiligen Organs kommen z.B. eine eingeschränkte freie Beweglichkeit (Darm). Derartige 0Adhäsionen von Organen mit dem sie umgebenden Gewebe sind vor allem für die betroffenen Patienten sehr schmerzhaft und können einen weiteren operativen Eingriff zur Beseitigung der Adhäsion zur Folge haben. Derzeit sind verschiedene Methoden und Materialien bekannt, die ein Auftreten von Adhäsionen nach einem operativen Eingriff reduzieren sollen, so z.B. aus der WO 02/09789. Allerdings ist es mit Unsicherheiten verbunden, den Zustand der Organe nach der Operation hinsichtlich möglicher Adhäsionen zu überwachen, da die inneren Organe bzw. das Körpergewebe nicht wie ein Knochen nach einer Operation mittels eines einfachen bildgebenden Verfahrens wie der Röntgenuntersuchung überwacht werden kann. Eine Methode, die ein nicht invasives Sichtbarmachen der inneren Organe sowie von Körpergewebe ermöglicht, ist die Ultraschalldetektion, die jedoch nur ein sehr unklares und nur für sehr geübte Fachleute aussagekräftiges Bild hinsichtlich der unerwünschten Adhäsion des Gewebes ermöglicht. Alternative Verfahren stellt die Überwachung des Tissue Growth Factors (TGF) in mehreren Blutproben sowie die Analyse der Drainageflüssigkeit nach bestimmten Botenstoffen dar. Diese beiden Verfahren sind allerdings in der Literatur sehr umstritten, da sich insbesondere nach operativen Eingriffen das Blutbild des Patienten ohnehin sehr verändert und dadurch die Ergebnisse der Blut- bzw. Drainageflüssigkeitsuntersuchungen mit einem sehr hohen Unsicherheitsfaktor belastet sind.

Ein weiteres Problem bei vielen operativen Eingriffen, bei denen ein nicht röntgensichtbares Implantat in den Körper des Patienten eingebracht wird, ist die postoperative Kontrolle der Ortsbeständigkeit des in einer aufwändigen Operation räumlich sehr akkurat platzierten Implantats. In vielen Fällen ist eine Ortsstabilität des Implantates für den Erfolg des Eingriffes unerlässlich. Beispielsweise ist es für die Funktionalität eines Herniennetzes erforderlich, dass es mittig über dem vorhandenen Bruch bleibt, auch wenn es nur mit minimalsten Fixierungsmitteln am Körpergewebe befestigt werden soll, um das umliegende Gewebe nicht zusätzlich zu traumatisieren.

Im medizinischen Bereich ist es üblich, Gegenstände in den Körper einzuführen, in dem zunächst ein Trokar eingeführt wird, durch den hindurch anschließend der in den Körper einzuführende Gegenstand verschoben wird. Bei dem Gegenstand kann sich um ein resorbierbares Trägermaterial, oder ein Implantat handeln, das endoskopisch eingeführt wird, wie z.B. Implantate zum Verschluss von Narbenhernien. Der Erfolg einer solchen Behandlung oder Implantierung hängt entscheidend davon ab, dass der Trokar in einer genau vorgegebenen Position innerhalb des Körpers angeordnet werden soll. Die Position des Trokars kann beim Einbringen durch ein Endoskop kontrolliert werden, was jedoch außerordentlich aufwendig und schwierig ist und zwar sowohl hinsichtlich der benötigten Apparaturen als auch hinsichtlich der benötigten Zeit sich in der endoskopischen Sichtweite zu orientieren.

Eine Möglichkeit ist, die Lage des in den Körper eingeführten Trokars durch herkömmliche computertomographischen Abbildungsverfahren zu kontrollieren, um die Lage der Hohlnadel im Raum und relativ zu dem Körper feststellen können und damit dem Operateur die Möglichkeit zu geben, den Trokar kontrolliert in den Körper einzubringen. Diese Positionierungsmethode bedarf allerdings eines erheblichen apparativen Aufbaus.

Die WO 02/096474 A1 betrifft einen Knochenzement auf Acrylatbasis, der röntgensichtbare Metallpartikel mit einem Durchmesser zwischen 1 und 100 µm enthält.

Aus der WO 01/56499 A1 geht ein flächiges Implantat mit einer flexiblen Trägerstruktur aus Polymer und röntgensichtbaren Elementen wie beispielsweise Kupfer, Silber oder Gold hervor, wobei die röntgensichtbaren Elemente eine Größe von 0,1 bis 50 mm aufweisen können.

Aufgabe der Erfindung ist es daher Mittel und Verfahren zu finden, die die postoperative Überwachung von nicht röntgensichtbaren Implantaten sowie die Überwachung des Heilungsprozesses von außen, d.h. ohne invasive Eingriffe, zuverlässig über einen längeren Zeitraum ermöglicht, ohne dass der Patient zusätzlichen Belastungen ausgesetzt ist und die mit üblichen vorhandenen apparativen Mitteln erfolgen kann.

Die Aufgabe wurde gelöst durch ein mindestens teilweise resorbierbares Trägermaterial mit antimikrobiell wirkenden Metall-, insbesondere Silberpartikeln von 0,2 µm bis 10 µm Größe zur Anwendung in einem menschlichen oder tierischen Körper. Das Material hat den Vorteil, dass es zunächst durch die antimikrobiellen Eigenschaften der Metallpartikel zur Reduzierung des Infektionsrisikos an der Einsatzstelle beiträgt und durch die Resorbierbarkeit des Trägermaterials nach einem gewissen Zeitraum mindestens teilweise abgebaut, resorbiert und aus dem Körper ausgeschieden werden kann. Das Trägermaterial ist vorzugsweise vollständig resobierbar. Als Metalle für die Partikel eignen sich auch Gold und Kupfer. Silber ist bevorzugt. Das erfindungsgemäße Material ist sehr vielseitig einsetzbar und kann sowohl alleine als auch in Kombination mit einem Implantat zum Einsatz kommen. Das erfindungsgemäße Material kann in der Diagnostik eingesetzt werden, um zu untersuchen, ob ein Implantat oder das Material selbst verrutscht ist, eine Verwachsung stattgefunden hat oder, in Kombination mit einer Bildauswertung, wie weit das Material bereits resorbiert ist. Unmittelbar bei operativen Eingriffen kann es zur apparativ einfachen Positionskontrolle des Implantats mit Trägermaterial oder des Trägermaterials alleine eingesetzt werden.

Das erfindungsgemäße resorbierbare Trägermaterial umfasst Silberpartikel der Größe 0,2 µm bis 10 µm, wobei sich diese einzelnen Partikel auch zu größeren Aggregaten zusammenlagern können und dann in Größen von bis zu 200 µm im Trägermaterial vorliegen können. Nach vollständiger Resorption des erfindungsgemäßen Trägermaterials verbleiben die Metallpartikel im Organismus, was jedoch aufgrund der geringen Menge und der vollkommen diffusen räumlichen Verteilung keine unerwünschten Nebenwirkungen oder Detektionsfehler bei späteren diagnostischen Verfahren verursachen kann.

Bei dem erfindungsgemäßen Trägermaterial handelt es sich in einer Ausführungsform um ein plastisch formbares Material. Bei dem Material handelt es sich vorzugsweise um eine Paste, ein Gel oder um eine Flüssigkeit. Auch ein versprühbares aggregierendes Pulver, insbesondere in Form einer Dispersion ist geeignet. Das flüssige Material kann sehr einfach auch mittels minimal invasiver Verfahren an jede gewünschte Stelle im Körpers gebracht werden. Es kann vorteilhafterweise durch Aufsprühen auf schwer zugängliche Stellen aufgetragen werden. Ein Gel bietet gegenüber der Flüssigkeit den Vorteil, dass es nach dem Auftragen vor allem auf gewölbten Flächen nicht so leicht abläuft, sondern am Applikationsort verbleibt.

In einer anderen Ausführungsform handelt es sich bei dem Trägermaterial um ein festes Material, vorzugsweise um ein Pulver, eine Folie und/oder eine Membran. Je nach Anwendungsgebiet ist es möglich, dass jeweils geeignetste Material auszuwählen, um einerseits das Material auf sensiblen Organoberflächen ohne Beeinträchtigungen für das Organ aufmodellieren und andererseits beispielsweise feste Implantate dauerhaft mit einer stabilen Form des erfindungsgemäßen Materials insbesondere in Form einer Folie versehen und implantieren zu können.

Mit Vorteil ist das erfindungsgemäße Material frei von jeglicher mechanischer Funktion und weist insbesondere nach dem Einbringen in den Körper keinerlei tragende oder stützende mechanische Funktionen auf. Diese mechanischen Funktionen bleiben gegebenenfalls im Körper verbleibenden Implantaten vorbehalten, wohingegen das erfindungsgemäße Material aufgrund seiner Resorbierbarkeit in einer besonderen Ausführungsform weniger für eine andauernde tragende oder stützende Funktion vorgesehen ist.

Das Trägermaterial entsprechend der vorliegenden Erfindung kann in einer besonderen Ausführungsform auch Verstärkungen durch einzelne oder verarbeitet Fasern aufweisen und/oder mit einem weiteren, vorzugsweise textilen Implantat und/oder einem geschäumten Material, insbesondere mit einem gewirkten oder gewebten Netz, kombiniert sein. Dadurch erhält das Material eine höhere Stabilität und partielle starke mechanische Beanspruchungen durch Reiben o.ä. führen nicht unmittelbar zu einem Schaden am Material. Bei den Verstärkungen kann es sich um Netze, Vliese und/oder Folien, insbesondere aus Polyurethan (PUR), handeln.

Mit Vorteil ist das Material direkt auf die unterschiedlichsten Wunden und/oder Organe aufbringbar. Dabei ist je nach Applikation, insbesondere beim Einsatz ohne zusätzliche Verstärkungen, und der jeweils erforderlichen Stabilität eine belastbare oder weniger belastbare Ausführung des Materials von Vorteil. In einer besonderen Ausführungsform ist die Stabilität und damit auch die Resorptionszeit des Materials durch physikalische, insbesondere thermische, Vernetzung innerhalb des Materials einstellbar.

Die Resorptionsdauer des Materials ist einstellbar und kann je nach Form des eingesetzten Materials und nach Art des Eingriffes sowie des Applikationsortes mehr oder weniger lang sein. Vorteilhafterweise beträgt die Resorptionsdauer in vivo 30 bis 220 Tage, insbesondere 60 bis 120 Tage, vorzugsweise 60 bis 90 Tage. Dabei ist die bevorzugte Resorptionsdauer eines Gels mit ca. 20 Tagen geringer als die mögliche Resorptionsdauer einer Membran oder Folie von 60 bis 120 Tagen oder die eines Pulvers von mehr als 90 Tagen.

Vorteilhafterweise ist der Resorptionsgrad des Materials jederzeit mittels eines physikalischen bildgebenden Verfahrens und einer entsprechenden Bildverarbeitung von außen detektierbar. Dies bringt den Vorteil, dass zur Diagnose kein operativer Eingriff oder eine für den Organismus belastende Untersuchung nötig ist.

Das erfindungsgemäße Trägermaterial enthält mit Vorteil mindestens eine der Komponenten aus der Gruppe bestehend aus Polyethylenglykol (PEG), Polyhydroxybuttersäure (PHB), Carboxymethylcellulose (CMC) Polyvinylalkohol (PVA), Dextranaldehyd, Chitosan, Glycolid, Lactid, Trimethylencarbonat, Epsiloncaprolacton und Dioxan. In einer bevorzugten Ausführungsform besteht das erfindungsgemäße Trägermaterial aus einer Membran, einem Gel oder einer Flüssigkeit aus einem CMC/PVA-Gemisch. Diese Materialmischung hat sich als besonders gut verarbeitbar und applizierbar erwiesen. Das Gewichtsverhältnis von CMC/PVA liegt vorzugsweise im Bereich von 1:1 bis 1:100, insbesondere 1:10 bis 1:50.

Bei den Metallpartikeln des Trägermaterials kann es sich um lösliche Partikel handeln. Es ist auch möglich, dass es sich bei den Metallpartikeln um unlösliche Partikel handelt. Je nach Verweildauer des Materials im Körper und Art des Eingriffes bzw. des Infektionsrisikos der Operationswunde ist es von Vorteil, entweder lösliche und/oder leichter verbreitbare Partikel oder unlösliche und/oder ortsfeste Partikel einzusetzen. In einer weiteren Ausführungsform kann auch eine Mischung aus löslichen und/oder leichter verbreitbaren Partikeln und unlöslichen und/oder ortsfesten Partikeln eingesetzt werden, um die Vorteile beider Partikelformen zu nutzen.

Erfindungsgemäß handelt es sich bei den Silberpartikeln vorteilhafter Weise um mindestens ein Material aus der Gruppe umfassend metallisches Silber, Silberkomplexe, insbesondere Silbercitratkomplexe, und Silberverbindungen. Dabei sind Silbersalze ganz besonders bevorzugt. Silberkomplexe, insbesondere Silbercitratkomplexe, können auch kovalent an Polymere gebunden sein. Entsprechendes gilt auch für die anderen Metalle.

Die Metallpartikel können im Trägermaterial gleichmäßig oder statistisch verteilt sein. Für besondere Zwecke können die Partikel auch an bestimmten Stellen angereichert sein, z.B. an der Oberfläche von festem Trägermaterial.

Gemäß einer Ausführungsform sind die Metallpartikel nicht nur als Schicht auf der Oberfläche des Trägermaterials, sondern auch oder ausschließlich statistisch über das gesamte Volumen des Trägermaterials homogen verteilt. Dadurch ist gewährleistet, dass während eines langsamen Abbaus des Materials ständig eine antimikrobiell aktive Oberfläche bis zu vollständigen Resorption des Materials vorliegt.

In einer anderen Ausführungsform können die Silberpartikel in einer bestimmten geometrischen Anordnung im Trägermaterial, insbesondere in einer Membran, vorliegen. Dies ermöglicht bei einer Detektion des Materials, insbesondere einer Membran, die Feststellung, ob die Membran in der gewünschten, vorzugsweise ebenen, Form am Zielort angebracht wurde oder ob die Membran eventuell übereinandergeschlagen oder in Falten vorliegt. Es ist weiterhin möglich, dass die Silberpartikel als Schicht im Trägermaterial vorliegen.

Der Anteil an Metallpartikeln im Trägermaterial hängt von der Raumform und der gewünschten Verteilung im Material ab und beträgt, insbesondere in einem Material in Form eines Gels oder einer Paste, bevorzugt bis zu 1 Gew.-% und besonders bevorzugt 0,2 bis 0,8 Gew.-%.

Bei einer definierten Oberfläche des Materials, insbesondere bei einem Material in Form einer Membran, kann eine Menge an Metall an der Oberfläche der Membran von vorzugsweise ca. 10 µg pro cm² vorliegen. Die Menge an Metall pro cm² hängt wie der prozentuale Anteil des Metalls stark vom jeweiligen Material und dessen Einsatzgebiet ab. Bei flüssigen Materialien, die keine Körperflüssigkeit mehr aufnehmen werden, reicht ein geringerer Anteil an Metall aus, wohingegen bei trockenen Materialien, welche im Körper vorzugsweise Flüssigkeit aufnehmen, ein höherer Anteil an Metall erwünscht ist.

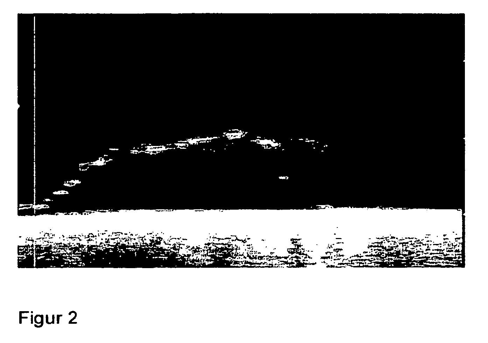

Vorteilhafter Weise ist das erfindungsgemäße silberpartikelhaltige Material, insbesondere im Körper, mittels mindestens einer physikalischen, vorzugsweise in Echtzeit, bildgebenden Methode detektierbar. Bei dieser Methode kann es sich um Ultraschall, Magnetic Resonance Imaging (MRI), Optical Coherence Tomography (OCT), Computertomographie (CT) und Röntgenstrahlen handeln. Mittels dieser bildgebenden Verfahren ist es möglich, ohne weitere für den Körper belastende zusätzliche Eingriffe, das während einer Operation im Körper platzierte Material und seine Lage zu detektieren. Vorzugsweise ist bei der Detektion die Detektionsebene, insbesondere die Bildebene einer Ultraschallaufnahme, senkrecht zur Ebene des implantierten Materials ausgerichtet (Figur 2).

Mit Vorteil ist das erfindungsgemäße Material in bewegtem Zustand detektierbar. Die Detektion des Materials mittels einer der zuvor genannten bildgebenden physikalischen Methoden erfolgt vorzugsweise in einem bewegten Zustand des Materials.

In einer anderen Ausführungsform ist die relative Beweglichkeit mindestens zweier getrennt voneinander vorliegender Materialien zueinander mittels mindestens einer physikalischen bildgebenden Methode, insbesondere mittels Ultraschall, MRI, OCT, CT und Röntgenstrahlung, detektierbar. Die Detektierbarkeit des Materials in bewegtem Zustand ermöglicht ein leichteres Erkennen von Verwachsungen von Körpergewebe gegenüber einer Detektion des ruhenden Materials im Körper. Besonders vorteilhaft ist die Detektierbarkeit im Fall des Vorliegens von zwei separaten am Körpergewebe fixierten Materialien, vorzugsweise Membranen oder Folien, wobei die Bewegung der beiden Materialen relativ zueinander detektiert wird. Damit ist eine Detektion von Verwachsungen möglich, da eine Bewegung der beiden Materialien relativ zueinander bei einer Verwachsung nicht mehr festzustellen wäre. Das Vorliegen einer Bewegung zeigt eine positive Wundheilung ohne Adhäsionen.

Mit Vorteil liegt das erfindungsgemäße Trägermaterial in einer Form vor, die direkt, gegebenenfalls nach einem Zuschnitt auf die jeweiligen Verhältnisse, auf eine Operationswunde und/oder auf angrenzendes Gewebe applizierbar ist. Dabei liegt das Material vorteilhafter Weise bereits in steriler Form vor. Es ist auch möglich, dass das Material in unterschiedlich großen und/oder vorgeformten Stücken vorliegt, die direkt auf unterschiedliche Wunden oder Organe aufbringbar sind.

Die vorliegende Erfindung umfasst auch ein medizintechnisches Produkt, welches ein mindestens teilweise resorbierbares Trägermaterial mit Metallpartikeln von 0,2 bis 10 µm Größe enthält, welches im menschlichen oder tierischen Körper einsetzbar ist. Dieses medizintechnische Produkt kann ein Implantat umfassen, welches auf mindestens einer Außenseite das Trägermaterial mit Metallpartikeln aufweist. Dadurch ist das Produkt mittels verschiedener bildgebender physikalischer Methoden während und nach einem operativen Eingriff leicht detektierbar bis zur vollständigen Resorption des Trägermaterials in einer Zeit je nach Art des Produktes und der Menge des applizierten bzw. angebrachten Trägermaterials.

Die Erfindung umfasst weiterhin die Bereitstellung eines mindestens teilweise resorbierbaren Trägermaterials mit Metallpartikeln der Größe 0,2 bis 10 µm zur Anwendung im menschlichen oder tierischen Körper. In einer Ausführungsform wird das Trägermaterial zum Abdecken einer Operationsstelle vor dem Verschluss der Wunde bereitgestellt.

In einer weiteren Ausführungsform wird das metallpartikelhaltige mindestens teilweise resorbierbare Trägermaterial zur postoperativen Positionsbestimmung der mittels eines Trägermaterials markierten inneren Wunde oder eines Implantates sowie zur Detektion von Adhäsionen im menschlichen oder tierischen Körper bereitgestellt.

Das erfindungsgemäße Material kann Metallpartikel in einer Konzentration enthalten, so dass es die Funktion eines Kontrastmittels, ähnlich einem Kontrastmittel bei Röntgenuntersuchungen, erfüllen kann. Es kann insbesondere in der Diagnostik und bei postoperativen Untersuchungen mittels Ultraschall eingesetzt werden, um die Funktionalität eines Kontrastmittels zu erfüllen.

Die Aufgabe der Erfindung wird weiterhin gelöst durch ein Verfahren zur Detektion eines metallpartikelhaltigen Trägermaterials im menschlichen oder tierischen Körper, wobei das metallpartikelhaltige Trägermaterial zunächst während einer Operation implantiert wird und nach der Operation mittels einer physikalischen bildgebenden Methode detektiert wird. Vorzugsweise liegt das Trägermaterial dabei in Kombination mit einem Implantat, vorzugsweise einem Herniennetz und/oder einem Prolapsnetz, vor. Das Implantat kann teilresorbierbar sein. Das Einführen des Trägermaterials mit oder ohne Implantat kann subkutan und/oder endoskopisch mittels eines Trokars und insbesondere mittels einer Reduzierhülse durchgeführt werden. Auch eine navigierte Positionierung des Trägermaterials mit einem Strahlungssender und einem Strahlungsempfänger ist möglich.

Dabei kann das Verfahren einerseits zur operativen Kontrolle der Position des Implantats mit Trägermaterial oder des reinen Trägermaterials, vorzugsweise in Form einer Membran oder eines Gels, eingesetzt werden. Auch ein selbst-aggregierendes (selbst-fusionierendes) Pulver ist geeignet. Es ist auch möglich, eine postoperative Positionskontrolle oder Detektion des Resorptionsgrades des Trägermaterials mittels des erfindungsgemäßen Verfahrens durchzuführen.

Mit Vorteil wird das Material in bewegtem Zustand detektiert. Durch die Bewegung entsteht ein Dopplereffekt, der physikalisch messbar ist und mittels bildgebender Methoden dargestellt werden kann.

Durch das erfindungsgemäße Verfahren kann die postoperative Ortsfestigkeit von fixierten Implantaten mit dem silberpartikelhaltigen Trägermaterial detektiert werden bzw. ein Verrutschen oder Ablösen eines Implantates vom gewünschten Applikationsort frühzeitig erkannt werden. Es ist auch möglich, das erfindungsgemäße Trägermaterial auf einer verklebten Wunde aufzutragen und zu detektieren, um damit zu überprüfen, ob der Wundverschluss, insbesondere mittels eines Gewebeklebers, ein dauerhafter ist oder ob die verklebte Wunde und damit auch eine Schicht des erfindungsgemäßen silberpartikelhaltigen Trägermaterials auf der Wunde wieder aufgerissen ist und damit auch im detektierten Bild aufklafft.

Weiterhin kann mittels des erfindungsgemäßen Verfahrens auch eine Adhäsion detektiert werden, in dem ein oder mehrere Trägermaterialien mit Metallpartikeln mit oder ohne Implantat implantiert werden und anschließend die Bewegung des Trägermaterials und des umgebenden Gewebes relativ zueinander ermittelt werden. In einer anderen Ausführung des Verfahrens kann die Bewegung zweier am Gewebe fixierter Trägermaterialien relativ zueinander ermittelt werden. Damit können Adhäsionen bereits im Anfangsstadium erkannt werden, indem die relative Bewegung des mindestens einen Trägermaterials abnimmt.

Beim erfindungsgemäßen Verfahren hat es sich als vorteilhaft erwiesen, dass die Detektion des Materials senkrecht zur Ebene des Materials erfolgt. Die Darstellung bzw. Wiedergabe der Messdaten erfolgt in Form eines mindestens zweidimensionalen Bildes.

Die Erfindung umfasst auch ein Verfahren zur Detektion von postoperativen Verwachsungen im menschlichen oder tierischen Körper, wobei die Beweglichkeit mindestens eines während eines operativen Eingriffs implantierten silberpartikelhaltigen Trägermaterials, vorzugsweise einer Membran oder eines Gels, relativ zum umgebenden Körpergewebe oder zu einem weiteren Trägermaterial mittels mindestens einer physikalischen bildgebenden Methode in zeitlichen Abständen gemessen wird. Bei der bildgebenden Methode handelt es sich insbesondere um Ultraschall, MRI, OCT, CT und Röntgenstrahlung, die ein mindestens zweidimensionales Bild entweder eines Schnittes durch das Trägermaterial oder in der Ebene des Trägermaterials generiert.
In einer Ausführungsform des Verfahrens wird die Detektion der Beweglichkeit des Trägermaterials in bewegtem Zustand, insbesondere in einer Ebene senkrecht zu der des Trägermaterials, durchgeführt.

Durch die Bewegung mindestens eines, insbesondere zweier, Trägermaterialien im Körper entsteht ein Dopplereffekt, der mittels physikalischer bildgebender Methoden bildlich dargestellt werden kann. Durch diese Detektion, d.h. die Darstellung der Beweglichkeit oder Unbeweglichkeit des Materials ist es möglich, aufgrund einer reduzierten Beweglichkeit des implantierten Trägermaterials zum umgebenden Gewebe oder aus der reduzierten relativen Bewegung mindestens zweier Trägermaterialien zueinander, von denen vorzugsweise mindestens eines an das Körpergewebe fixiert wurde, eine beginnende Adhäsion zu lokalisieren. Dadurch können frühzeitig Gegenmaßnahmen eingeleitet werden, bevor Gewebeteile oder Organe durch Verwachsungen in ihrer Funktion eingeschränkt oder in ihrer Struktur nachhaltig geschädigt werden.

Vorzugsweise wird das Verfahren unmittelbar nach einer Operation und anschließend in unterschiedlichen Zeitabständen von 5 bis 10 Tagen, in den ersten Wochen nach der Operation und danach in ca. monatlichem Abstand, im ersten Jahr nach der Operation, durchgeführt, um eine Veränderung der relativen Beweglichkeit des silberpartikelhaltigen Trägermaterials zu messen und damit Adhäsionen zu lokalisieren.

Weitere Merkmale der Erfindung ergeben sich aus der nachfolgenden Beschreibung von bevorzugten Ausführungsformen und Beispielen. Hierbei können die einzelnen Merkmale der Erfindung allein oder in Kombination miteinander verwirklicht sein. Die beschriebenen Ausführungsformen dienen zur Erläuterung und zum besseren Verständnis der Erfindung und sind in keiner Weise einschränkend zu verstehen.

### Beispiele

Die Angabe ppm bezieht sich im folgenden immer auf die Menge Wasser, Membran oder Gel.

### 1. Herstellung einer Membran mit Silberpartikeln

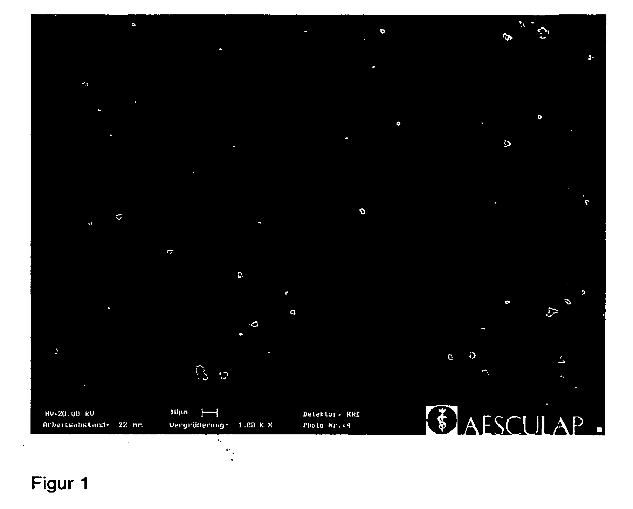

1428 ppm Silber pro Gramm Membran: 225,7 mg Silberpartikel der Größe 1-10 µm werden in 500 ml Wasser mit einem Ultra Turrax dispergiert und damit insgesamt 500 ml einer Silberpartikel haltigen Dispersion hergestellt. Anschließend werden 20 g Polyvinylalkohol (PVA)-Granulat zugegeben und die Mischung wird für ca. 12 Stunden auf 95 °C erhitzt, um den PVA zu lösen. Danach wird die Mischung nochmals mit dem Ultra Turrax dispergiert und mit 400 ml einer 4 Gew.-%igen wässrigen Carboxymethylcellulose(CMC)-Lösung vermischt und das Gemisch auf einer Rakel zu einer ca. 50 µm dicken Membran verarbeitet. Dabei wird ein Silbergehalt von ca. 10 µg/cm², was einer Konzentration von 1428 ppm bzw. 0,001428 Gew.-% Silber in der Membran entspricht, erzielt. Die ansonsten transparente Membran verfärbt sich durch Zugabe des Silbers leicht grau. In Rasterelektronenmikroskop-Bild (Figur 1) ist die statistische Verteilung des Silbers gut zu erkennen.

### 2. Herstellung einer Membran mit Silberdihydrogencitrat

20 Gew.-% PVA-Granulat werden in Wasser gegeben und mit unterschiedlichen Konzentrationen (30, 300, 1428 ppm - bezogen auf Gramm Membran) an Silberdihydrogencitrat versetzt. Die Mischung wird für ca. 12 Stunden erhitzt. 400 ml der Mischungen werden mit 500 ml einer 4 Gew.-%igen wässrigen CMC-Lösung vermischt und auf einer Rakel zu einer Membran verarbeitet (30 min.; 70 °C). Die PVA-Lösung ändert dabei ihre Farbe und wird trübe.

### 3. Herstellung einer Membran mit Silber-Hybridpartikeln

20 Gew.-% PVA-Granulat werden in 350 ml Wasser gemischt und 13,42 ml einer Lösung an Silberhybridpartikeln (1,5 Gew.-% reduzierte 1-2 nm große Silberpartikel in Lösung mit einer Hülle aus verzweigten amphiphilen modifizierten Polyethyleniminen) zugegeben (Membran mit 1428 ppm Silber). Das PVA wird bei 95 °C gelöst (400 ml) und 500 ml einer 4 Gew.-%igen wässrigen CMC-Lösung zugegeben. Diese Mischung wird auf einer Rakel zu einer Membran verarbeitet.

### 4. Wirksamkeit einer Silberpartikel haltigen Membran nach 1.

Die antibakterielle Wirkung wurde durch Versuche mit E.coli und S.aureus ATCC25123 untersucht. Durch folgenden Kurzzeittest wird die Wirkung der Membran direkt nach Applikation in den Körper gezeigt: Die Membran wird 2 Stunden in 50 ml Bakteriensuspension (n= 1×10⁷ pro ml) in PBS-Puffer bei 37 °C in einem Schüttelinkubator (U= 150 rpm) inkubiert. Nach dreimaligem Waschen mit PBS Puffer wurde die Membran nochmals eine Stunde in PBS Puffer inkubiert. Danach wurde die Membran in eine Petrischale überführt und fester Agar darüber gegeben. Nach einem Tag wurde das Bakterienwachstum überprüft. Die Membran (10 µg Silber/cm²) zeigte keinerlei bakterielles Wachstum. Durch einen weiteren Test wurde die Wirksamkeit der Membran bei längerer Anwendung im Bauchraum simuliert, bei der ständig peritoneale Flüssigkeit ausgetauscht wird. Um diese Situation zu simulieren, wurde die Membran 7 Tage inkubiert und dabei täglich mit jeweils frischem PBS-Puffer gewaschen. Nach dieser Simulation zeigt die Membran immer noch antibakterielle Wirksamkeit, d.h. kein Bakterienwachstum.

### 5. Wirksamkeit einer Silberdihydrogencitrat haltigen Membran nach 2.

Im oben beschriebenen Kurzzeittest zeigte eine Membran mit Silberdihydrogencitrat sowohl gegen E.coli als auch gegen S.aureus ab einer Konzentration von 300 ppm eine antimikrobielle Wirkung, d. h. es war kein Bakterienwachstum festzustellen.

### 6. Wirksamkeit einer Silberhybridpartikel haltigen Membran nach 3.

Im oben beschriebenen Kurzzeittest zeigte eine Membran mit einer Konzentration von 1428 ppm Silberhybridpartikeln (Reduziertes Silber mit verzeigten amphiphilen modifizierten Polyethyleniminen sowohl gegen E.coli als auch gegen S.aureus eine antimikrobielle Wirkung, d. h. es war kein Bakterienwachstum festzustellen.

### 7. Release Tests mit einer Silberpartikel haltigen Membran nach 1.

Das Freisetzungsverhalten der Antibakteriellen Stoffe aus der Membran wurde in destilliertem Wasser über einen Zeitraum von 7 Tagen untersucht. Die Membran wurde in 50 ml Wasser bei 37 °C inkubiert. Es wurden jeweils Membranen mit und ohne antibakterielle Substanz untersucht und parallel die wässrige Phase sowie die Ausgangs-Silbersuspension mittels UV-Vis untersucht. Der Test zeigte, dass die Silberpartikel über den gesamten Zeitraum von 7 Tagen zu 100 % in der Membran verbleiben. Erst durch Auflösen der Membran bei 95 °C werden die Silberpartikel freigesetzt.
Die antibakterielle Aktivität in vivo wird dabei durch die kontinuierliche Abgabe von Silberionen gewährleistet.

### 8. Release Tests mit einer Silberdihydrogencitrat haltigen Membran nach 2.

Das Verhalten der Membran mit 1428 ppm Silberdihydrogencitrat wurde ebenso untersucht. Membranstücke wurden bei 37 °C inkubiert und nach 8 Tagen bei 95 °C aufgelöst. Nach 5 Minuten bei 37 °C gingen zwischen 50 und 60 Gew.-% des Silberdihydrogencitrats aus der Membran in Lösung. In den weiteren 7 Tagen dagegen blieb der Wert konstant. Erst nach Auflösen der Membran kann das übrige (insgesamt zu ca. 90 Gew.-%) Silberdihydrogencitrat nachgewiesen werden.

### 9. Release Tests mit einer Silberhybridpartikel haltigen Membran nach 3.

Die Untersuchung der Silberhybridpartikel haltigen Membran ergab keine Freisetzung der Silberhybridpartikel innerhalb von 6 Tagen. Nach 7 Tagen bei 37 °C konnten 25 Gew.-% der Silberhybridpartikel in der wässrigen Phase nachgewiesen werden. Nach ca. 7 Tagen beginnt die Membran sich langsam aufzulösen. Nach dem Auflösen der Membran bei 95 °C können die Silberhybridpartikel zu 100 % nachgewiesen werden.

### 10. Detektion einer Silberpartikel haltigen Membran nach 1 mittels Ultraschall

Die Ultraschallaufnahmen (Figur 1 und 2) wurden mit einem Ultraschallgerät bei 8 Mhz aufgenommen. Sie dienen dazu, herauszufinden, ob die Membran mit den darin enthaltenen Silberpartikeln in Ultraschallbildern erkennbar und damit im Körper nach der Operation zu lokalisieren sind. Für die Aufnahmen wurde die Membran auf einem Styroporblock fixiert und unter Wasser getaucht. Es wurden verschiedenen Konzentrationen an Silberpartikeln der Größe 5-10 mm untersucht: 300, 600, 900, 1428 und 31250 ppm. Ab einer Konzentration von 600 ppm waren die Silberpartikel gut im Ultraschallbild zu erkennen.

### 11. Detektion von Silberpartikeln in einem Hydrogel zwischen Gewebeschichten

In einem Hydrogel werden 1428 ppm (bezogen auf Gramm Gel) Silberpartikel der Größe 5-10 µm homogen verteilt. Das Hydrogel wurde auf ein Schweineschnitzel als Körpergewebeersatz in einer dünnen Schicht appliziert und mit einem weiteren Schnitzel bedeckt. Diese Anordnung wurde dann unter Wasser getaucht und mittels Ultraschall wie unter 10. untersucht. Damit wird die einfache Detektion des Silberpartikelhaltigen Trägermaterials in vivo gezeigt. Die Ultraschallaufnahmen zeigten deutlich die Silberpartikel und bei bewegen des Ultraschallkopfes waren deutlich die aufblitzenden Partikel zu erkennen.

### Figuren

Figur 1 zeigt eine Rasterelektronenmikroskop-Aufnahme der Aufsicht auf eine 1428 ppm Silberpartikel pro Gramm Membran haltigen PVA/CMC-Membran nach Beispiel 1. Die statistische Verteilung des Silbers ist gut zu erkennen.
Figur 2 zeigt eine Ultraschallaufnahme einer PVA/CMC-Membran mit 1428 ppm Silberpartikel pro Gramm Membran (vgl. Beispiel 10). Die Membran wurde mit einer Nadel an einem Ende auf einem Styroporblock befestigt, der als heller Untergrund erkennbar ist, und in Wasser getaucht. Senkrecht dazu wurde mit 8 MHz ein Querschnitt der Membran im Ultraschall vermessen. Die Silberpartikel in der Membran sind als helle Flecken erkennbar.

## Patentansprüche

1. Mindestens teilweise resorbierbares Trägermaterial mit antimikrobiell wirkenden Metallpartikeln, insbesondere Silberpartikeln, von 0,2 µm bis 10 µm Größe zur Anwendung im menschlichen oder tierischen Körper.

2. Material nach Anspruch 1, **dadurch gekennzeichnet, dass** es sich um ein plastisch formbares Material, vorzugsweise in mindestens einer Form aus der Gruppe umfassend ein Gel, eine Dispersion und eine Flüssigkeit, handelt.

3. Material nach Anspruch 1, **dadurch gekennzeichnet, dass** es sich um ein festes Material, vorzugsweise um mindestens eine Form aus der Gruppe umfassend ein Pulver, eine Folie und eine Membran, handelt.

4. Material nach einem der vorhergehenden Ansprüche, **dadurch gekennzeichnet, dass** es frei von einer mechanischen Funktion ist, insbesondere im menschlichen oder tierischen Körper keine mechanische, tragende oder stützende Funktion aufweist.

5. Material nach einem der vorhergehenden Ansprüche, **dadurch gekennzeichnet, dass** es eine Resorptionsdauer in vivo von 30 - 220 Tagen, insbesondere 60-120 Tagen, aufweist.

6. Material nach einem der vorhergehenden Ansprüche, **dadurch gekennzeichnet, dass** das Trägermaterial mindestens eine der Komponenten aus der Gruppe umfassend CMC, PVA, Polyethylenglykol (PEG), Polyhydroxybuttersäure (PHB), Dextranaldehyd, Chitosan, und/oder aus der Gruppe umfassend Polymere und Copolymere von Glycolid, Lactid, Trimethylencarbonat, Epsiloncaprolacton und Dioxanon enthält und insbesondere aus einer CMC/PVA Membran oder Gel besteht.

7. Material nach einem der vorhergehenden Ansprüche, **dadurch gekennzeichnet, dass** es sich bei den antimikrobiell wirkenden Metallpartikeln um lösliche und/oder unlösliche Partikel handelt.

8. Material nach einem der vorhergehenden Ansprüche, **dadurch gekennzeichnet, dass** es sich bei den Silberpartikeln um mindestens ein Material aus der Gruppe umfassend metallisches Silber, Silber-Komplexe, insbesondere Silber-Citrat-Komplexe, und Silberverbindungen, vorzugsweise Silbersalze, handelt.

9. Material nach einem der vorhergehenden Ansprüche, **dadurch gekennzeichnet, dass** die antimikrobiell wirkenden Metallpartikel statistisch im Trägermaterial verteilt sind.

10. Material nach einem der vorhergehenden Ansprüche, **dadurch gekennzeichnet, dass** die antimikrobiell wirkenden Metallpartikel geordnet im Trägermaterial, insbesondere in einer Membran, vorliegen.

11. Material nach einem der vorhergehenden Ansprüche, **dadurch gekennzeichnet, dass** der Anteil an Metallpartikeln des Trägermaterials, insbesondere in Form eines Gels, einer Paste, eines Pulvers oder einer Membran bis zu 1 Gew.-%, vorzugsweise 0,2 - 0,8 Gew.-%, beträgt.

12. Material nach einem der vorhergehenden Ansprüche, **dadurch gekennzeichnet, dass** der Metallgehalt an der Materialoberfläche des Trägermaterials, insbesondere in Form einer Membran, 1 bis 30 µg pro cm ², vorzugsweise ca. 10 µg pro cm², beträgt.

13. Material nach einem der vorhergehenden Ansprüche, **dadurch gekennzeichnet, dass** es, vorzugsweise im Körper, mittels mindestens einer physikalischen, vorzugsweise in Echtzeit, bildgebenden Methoden, insbesondere Ultraschall, Magnetic Resonance Imaging (MRI), Optical Coherence Tomography (OCT) oder CT (Computertomographie) und Röntgenstrahlung detektierbar ist.

14. Material nach einem der vorhergehenden Ansprüche, **dadurch gekennzeichnet, dass** es in Bewegung detektierbar ist.

15. Material nach einem der vorhergehenden Ansprüche, **dadurch gekennzeichnet, dass** eine relative Bewegbarkeit mindestens zweier getrennt voneinander vorliegender Materialien zueinander mittels mindestens einer physikalischen bildgebenden Methode, insbesondere Ultraschall, MRI, OCT, CT und Röntgenstrahlung detektierbar ist.

16. Material nach einem der vorhergehenden Ansprüche, **dadurch gekennzeichnet, dass** es in einer unmittelbar auf eine Operationswunde und/oder angrenzendes Gewebe applizierbaren Form, insbesondere in steriler Form, vorliegt.

17. Medizintechnisches Produkt zur Anwendung im menschlichen oder tierischen Körper, enthaltend ein mindestens teilweise resorbierbares Trägermaterial mit antimikrobiell wirkenden Metallpartikeln der Größe 0,2 bis 10 µm nach einem der vorhergehenden Ansprüche.

18. Verwendung eines mindestens teilweise resorbierbaren Trägermaterials mit antimikrobiell wirkenden Metallpartikeln der Größe 0,2 µm bis 10 µm, insbesondere eines solchen nach einem der Ansprüche 1 bis 16 zur Herstellung eines medizinischen Produktes zur Anwendung im menschlichen oder tierischen Körper.

19. Verwendung nach Anspruch 18 zum Abdecken einer Operationsstelle vor dem Wundverschluss.

20. Verwendung nach Anspruch 18 zur postoperativen Detektion der Position einer mittels des Materials markierten, versorgten inneren Wunde und/oder eines Implantats und/oder zur Detektion von Adhäsionen im menschlichen oder tierischen Körper.

21. Verwendung eines antimikrobiell wirkende Metallpartikel haltigen Trägermaterials nach einem der Ansprüche 1 bis 16, in einem Verfahren zur Detektion im menschlichen oder tierischen Körper, wobei das metallpartikelhaltige Trägermaterial insbesondere in Kombination mit einem Implantat, vorzugsweise einem Herniennetz, zunächst während einer Operation implantiert wird und nach der Operation mittels einer physikalischen bildgebenden Methode detektiert wird.

22. Verwendung nach Anspruch 21, **dadurch gekennzeichnet, dass** das Material in bewegtem Zustand detektiert wird.

23. Verwendung nach Anspruch 21 oder 22, **dadurch gekennzeichnet, dass** die Detektion des Materials senkrecht zur Ebene des Materials erfolgt.

24. Verfahren zur Detektion von postoperativen Verwachsungen im menschlichen oder tierischen Körper, wobei die Beweglichkeit mindestens eines während eines operativen Eingriffs implantierten Metallpartikel haltigen Trägermaterials nach einem der Ansprüche 1 bis 16, vorzugsweise einer Membran, eines Pulvers, einer Paste oder eines Gels, relativ zum umgebendem Körpergewebe oder zu einem weiteren Trägermaterial mittels mindestens einer physikalischen bildgebenden Methode, insbesondere Ultraschall, MRI, OCT, CT und Röntgenstrahlung, in zeitlichen Abständen gemessen wird.

25. Verfahren nach Anspruch 24, **dadurch gekennzeichnet, dass** die Messung der Beweglichkeit des Trägermaterials in bewegtem Zustand, insbesondere in einer Eben senkrecht zu der des Trägermaterials, erfolgt.

26. Verfahren nach einem der Ansprüche 21 bis 25, **dadurch gekennzeichnet, dass** unmittelbar nach der Operation und danach in unterschiedlichen zeitlichen Abständen von 5 bis 10 Tagen in den ersten Wochen nach der Operation und anschließend in ca. monatlichen Abstand im ersten Jahr nach der Operation die relative Beweglichkeit des Materials gemessen wird.

## Claims

1. At least partially resorbable carrier material comprising antimicrobial metal particles, more particularly silver particles, of from 0.2 µm to 10 µm in size for use in the human or animal body.

2. Material according to Claim 1, **characterized in that** it is a plastically deformable material, preferably in at least one form from the group comprising a gel, a dispersion and a liquid.

3. Material according to Claim 1, **characterized in that** it is a solid material, preferably at least one form from the group comprising a powder, a film and a membrane.

4. Material according to any of the preceding claims, **characterized in that** it has no mechanical function, more particularly has no mechanical, carrying or supporting function in the human or animal body.

5. Material according to any of the preceding claims, **characterized in that** it has an in vivo resorption period of 30-220 days, more particularly 60-120 days.

6. Material according to any of the preceding claims, **characterized in that** the carrier material contains at least one of the components from the group comprising CMC, PVA, polyethylene glycol (PEG), polyhydroxybutyrate (PHB), dextran aldehyde, chitosan, and/or from the group comprising polymers and copolymers of glycolide, lactide, trimethylene carbonate, epsilon caprolactone and dioxanone, and more particularly consists of a CMC/PVA membrane or gel.

7. Material according to any of the preceding claims, **characterized in that** the antimicrobial metal particles are soluble and/or insoluble particles.

8. Material according to any of the preceding claims, **characterized in that** the silver particles are at least one material from the group comprising metallic silver, silver complexes, more particularly silver-citrate complexes, and silver compounds, preferably silver salts.

9. Material according to any of the preceding claims, **characterized in that** the antimicrobial metal particles are distributed randomly in the carrier material.

10. Material according to any of the preceding claims, **characterized in that** the antimicrobial metal particles are present in an organized manner in the carrier material, especially in a membrane.

11. Material according to any of the preceding claims, **characterized in that** the proportion of metal particles in the carrier material, especially in the form of a gel, a paste, a powder or a membrane, is up to 1% by weight, preferably 0.2-0.8% by weight.

12. Material according to any of the preceding claims, **characterized in that** the metal content on the material surface of the carrier material, especially in the form of a membrane, is from 1 to 30 µg per cm², preferably about 10 µg per cm².

13. Material according to any of the preceding claims, **characterized in that** it is detectable, preferably in the body, by means of at least one physical, preferably in real time, imaging method, more particularly ultrasound, magnetic resonance imaging (MRI), optical coherence tomography (OCT) or CT (computed tomography) and X-radiation.

14. Material according to any of the preceding claims, **characterized in that** it is detectable in motion.

15. Material according to any of the preceding claims, **characterized in that** relative mobility of at least two separate materials with respect to one another is detectable by means of at least one physical imaging method, more particularly ultrasound, MRI, OCT, CT and X-radiation.

16. Material according to any of the preceding claims, **characterized in that** it is present in a form which is directly applicable onto a surgical wound and/or adjacent tissue, more particularly in a sterile form.

17. Medical technology product for use in the human or animal body, containing an at least partially resorbable carrier material comprising antimicrobial metal particles of from 0.2 to 10 µm in size according to any of the preceding claims.

18. Use of an at least partially resorbable carrier material comprising antimicrobial metal particles of from 0.2 µm to 10 µm in size, more particularly such a material according to any of Claims 1 to 16, for producing a medical product for use in the human or animal body.

19. Use according to Claim 18 for covering a surgical site before wound closure.

20. Use according to Claim 18 for the post-operative detection of the position of a treated inner wound labelled using the material and/or of an implant and/or for the detection of adhesions in the human or animal body.

21. Use of an antimicrobial metal particle-containing carrier material according to any of Claims 1 to 16 in a method for detection in the human or animal body, wherein the metal particle-containing carrier material, especially in combination with an implant, preferably a hernia mesh, is first implanted during surgery and is detected after surgery by means of a physical imaging method.

22. Use according to Claim 21, **characterized in that** the material is detected in an animated state.

23. Use according to Claim 21 or 22, **characterized in that** the material is detected perpendicularly to the plane of the material.

24. Method for detecting post-operative adhesions in the human or animal body, wherein the mobility of at least one metal particle-containing carrier material according to any of Claims 1 to 16 that was implanted during a surgical procedure, preferably a membrane, a powder, a paste or a gel, relative to the surrounding body tissue or to a further carrier material is measured at time intervals by means of at least one physical imaging method, more particularly ultrasound, MRI, OCT, CT and X-radiation.

25. Method according to Claim 24, **characterized in that** the mobility of the carrier material in an animated state is measured, especially in a plane perpendicular to that of the carrier material.

26. Method according to any of Claims 21 to 25, **characterized in that** the relative mobility of the material is measured immediately after surgery and thereafter at varying intervals from 5 to 10 days during the first few weeks after surgery and subsequently at an interval of about a month during the first year after surgery.

## Revendications

1. Matériau support au moins partiellement résorbable contenant des particules métalliques à effet antimicrobien, en particulier des particules d'argent, d'une taille de 0,2 µm à 10 µm destiné à une utilisation dans le corps humain ou animal.

2. Matériau selon la revendication 1, **caractérisé en ce qu'**il s'agit d'un matériau plastiquement déformable, de préférence sous au moins une forme du groupe comprenant un gel, une dispersion et un liquide.

3. Matériau selon la revendication 1, **caractérisé en ce qu'**il s'agit d'un matériau solide, de préférence sous au moins une forme du groupe comprenant une poudre, une feuille et une membrane.

4. Matériau selon l'une quelconque des revendications précédentes, **caractérisé en ce qu'**il est exempt d'une fonction mécanique, en particulier ne présente pas de fonction mécanique, de support ou d'appui dans le corps humain ou animal.

5. Matériau selon l'une quelconque des revendications précédentes, **caractérisé en ce qu'**il présente une durée de résorption in vivo de 30-220 jours, en particulier de 60-120 jours.

6. Matériau selon l'une quelconque des revendications précédentes, **caractérisé en ce que** le matériau support contient au moins un des composants du groupe comprenant la CMC, le PVA, le polyéthylèneglycol (PEG), le poly(acide hydroxybutyrique) (PHB), l'aldéhyde de dextrane, le chitosane, et/ou du groupe comprenant les polymères et les copolymères de glycolide, de lactide, de triméthylènecarbonate, d'epsilon-caprolactone et de dioxanone et est en particulier constitué d'une membrane ou d'un gel de CMC/PVA.

7. Matériau selon l'une quelconque des revendications précédentes, **caractérisé en ce qu'**il s'agit, pour les particules métalliques à effet antimicrobien de particules solubles et/ou insolubles.

8. Matériau selon l'une quelconque des revendications précédentes, **caractérisé en ce qu'**il s'agit, pour les particules d'argent, d'au moins un matériau du groupe comprenant l'argent métallique, les complexes d'argent, en particulier les complexes d'argent-citrate, et les composés à base d'argent, de préférence les sels d'argent.

9. Matériau selon l'une quelconque des revendications précédentes, **caractérisé en ce que** les particules métalliques à effet antimicrobien sont réparties statistiquement dans le matériau support.

10. Matériau selon l'une quelconque des revendications précédentes, **caractérisé en ce que** les particules métalliques à effet antimicrobien se trouvent sous forme ordonnée dans le matériau support, en particulier dans une membrane.

11. Matériau selon l'une quelconque des revendications précédentes, **caractérisé en ce que** la proportion de particules métalliques du matériau support, en particulier sous forme d'un gel, d'une pâte, d'une poudre ou d'une membrane est de jusqu'à 1% en poids, de préférence de 0,2-0,8% en poids.

12. Matériau selon l'une quelconque des revendications précédentes, **caractérisé en ce que** la teneur en métaux à la surface du matériau support, en particulier sous forme d'une membrane, est de 1 à 30 µg pro cm², de préférence d'environ 10 µg pro cm².

13. Matériau selon l'une quelconque des revendications précédentes, **caractérisé en ce qu'**il peut être détecté, de préférence dans le corps, au moyen d'au moins un procédé physique d'imagerie, de préférence en temps réel, en particulier par ultrasons, par imagerie par résonance magnétique (IRM), par tomographie par cohérence optique (OCT) ou par CT (tomographie informatisée) et les rayons X.

14. Matériau selon l'une quelconque des revendications précédentes, **caractérisé en ce qu'**il est détectable en mouvement.

15. Matériau selon l'une quelconque des revendications précédentes, **caractérisé en ce qu'**une mobilité relative l'un par rapport à l'autre d'au moins deux matériaux séparés l'un de l'autre est détectable au moyen d'au moins un procédé physique d'imagerie, en particulier les ultrasons, l'IRM, l'OCT, la CT et les rayons X.

16. Matériau selon l'une quelconque des revendications précédentes, **caractérisé en ce qu'**il se trouve sous une forme pouvant être appliquée directement sur une plaie opératoire et/ou une zone adjacente, en particulier sous forme stérile.

17. Produit technico-médical destiné à une utilisation dans un corps humain ou animal, contenant un matériau support au moins partiellement résorbable contenant des particules métalliques à effet antimicrobien d'une taille de 0,2 µm à 10 µm selon l'une quelconque des revendications précédentes.

18. Utilisation d'un matériau support au moins partiellement résorbable contenant des particules métalliques à effet antimicrobien, d'une taille de 0,2 µm à 10 µm, en particulier un matériau selon l'une quelconque des revendications 1 à 16 pour la fabrication d'un produit médical destiné à une utilisation dans le corps humain ou animal.

19. Utilisation selon la revendication 18 pour le recouvrement d'un site opératoire, avant de fermer la plaie.

20. Utilisation selon la revendication 18 pour la détection post-opératoire de la position d'une plaie soignée interne marquée au moyen du matériau et/ou d'un implant et/ou pour la détection d'adhérences dans le corps humain ou animal.

21. Utilisation d'un matériau support contenant des particules métalliques à effet antimicrobien selon l'une quelconque des revendications 1 à 16, dans un procédé pour la détection dans le corps humain ou animal, le matériau support contenant des particules métalliques étant en particulier d'abord implanté pendant une opération en combinaison avec un implant, de préférence un filet à hernie, puis est détecté après l'opération au moyen d'un procédé physique d'imagerie.

22. Utilisation selon la revendication 21, **caractérisée en ce que** le matériau est détecté dans un état en mouvement.

23. Utilisation selon la revendication 21 ou 22, **caractérisée en ce que** la détection du matériau a lieu perpendiculairement par rapport au plan du matériau.

24. Procédé pour la détection de difformités postopératoires dans le corps humain ou animal, la mobilité d'au moins un matériau support contenant des particules métalliques implanté pendant une intervention opératoire selon l'une quelconque des revendications 1 à 16, de préférence une membrane, une poudre, une pâte ou un gel, étant mesurée par rapport au tissu environnant du corps ou par rapport à un autre matériau support au moyen d'au moins un procédé physique d'imagerie, en particulier les ultrasons, l'IRM, l'OCT, la CT et les rayons X, à des intervalles dans le temps.

25. Procédé selon la revendication 24, **caractérisé en ce que** - la mesure de la mobilité du matériau support a lieu dans un état en mouvement, en particulier dans un plan perpendiculaire à celui du matériau support.

26. Procédé selon l'une quelconque des revendications 21 à 25, **caractérisé en ce que** la mobilité relative du matériau est mesurée directement après l'opération et ensuite à des différents intervalles dans le temps de 5 à 10 jours au cours des premières semaines après l'opération et ensuite à un intervalle environ mensuel pendant la première année après l'opération.
